# EUROPEAN PATENT APPLICATION

(11) **EP 4 778 928 A1**
(43) Date of publication of application: **22.07.2026**
(21) Application number: 24864781.0
(22) Date of filing: 14.09.2024
(51) Int. Cl.: C07D 491/22, A61K 31/4745, A61P 35/00

(54) **EXATECAN HYDROXYLAMINE DERIVATIVE AND USE THEREOF**

(30) Priority: 15.09.2023 CN 202311192704
(71) Applicant: Hangzhou Adcoris Biopharma Co., Ltd, Hangzhou, Zhejiang 310052 (CN)
(72) Inventor: MIAO, Zhenwei, Hangzhou, Zhejiang 310052 (CN); HUANG, Yunsheng, Hangzhou, Zhejiang 310052 (CN); LI, Yaowu, Hangzhou, Zhejiang 310052 (CN)
(74) Representative: Elzaburu S.L.P.
(86) International application number: PCT/CN2024/119146
(87) International publication number: WO 2025/056066

(57) **Abstract**

The present invention relates to a method for preparing a series of exatecan derivatives containing a hydroxylamine structure substitution and pharmaceutically acceptable salts thereof, and the use thereof in the field of anti-tumor. These exatecan derivatives not only have a good activity of inhibiting the growth of tumor cells, but also have a good capability of permeating cell membranes or bystander effect, and can be used as monotherapy, combination therapy, or as toxin components of ADC for the treatment of tumors.

## Description

The present application claims priority from the Chinese patent application No. 2023111927049 filed on September 15, 2023, and the present application incorporates by reference the entire content of the above-mentioned Chinese patent application.

### TECHNICAL FIELD

The present disclosure relates to the field of biomedicine, and specifically relates to exatecan hydroxylamine derivatives and use thereof.

### BACKGROUND

DNA topoisomerases are a class of essential enzymes widely present in organisms. They affect DNA topology by regulating supercoiling, catenation, decatenation, and strand dissociation, and are mainly classified into topoisomerase 1 (Top1) and topoisomerase 2 (Top2). Compared with Top2 inhibitors, Top1 inhibitors have high efficacy and a broad antitumor spectrum, and have become important target enzymes for designing novel antitumor drugs. Meanwhile, the level of Top1 in various tumor cells such as colon cancer, cervical cancer, ovarian cancer, etc., is significantly higher than that in normal tissues, and its activity is greatly increased in S-phase tumor cells. Therefore, Top1-inhibiting drugs can selectively inhibit DNA replication in proliferating tumor cells, exhibiting good selectivity.

Camptothecin exhibits Top1 inhibitory activity, particularly strong inhibitory activity against the Top1-DNA complex. Topoisomerase 1 (Top1) is the primary target of camptothecin (CPT) and its analogs. Therefore, camptothecin demonstrates significant efficacy against various solid tumors such as gastric cancer, esophageal cancer, lung cancer, bladder cancer, etc., and is a broad-spectrum antitumor agent. Among them, irinotecan, topotecan, and belotecan have been approved for the treatment of various cancers in some countries. In addition, DX-8951 and SN38 have been successfully used as payloads for antibody-drug conjugates (ADCs) in targeted therapy of various solid tumors, and have attracted attention due to their remarkable efficacy. Currently, exatecan is characterized by strong toxic side effects, poor cell permeability, and poor stability in the blood system.

### SUMMARY

The present disclosure provides a series of novel exatecan derivatives, pharmaceutically acceptable salts, stereoisomers or prodrugs thereof, and use thereof in the field of anti-tumor. These exatecan derivatives not only have a good activity of inhibiting the growth of tumor cells, but also have a good capability of permeating cell membranes or bystander effect, and can be used as monotherapy, combination therapy, or as toxin components of ADC for the treatment of tumors.

One aspect of the present disclosure provides a compound of formula (I), or a pharmaceutically acceptable salt, a stereoisomer, or a prodrug thereof: 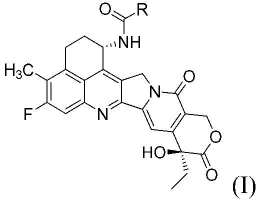 in the Formula, R is selected from 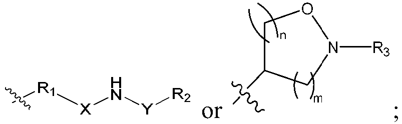
wherein, R₁ is selected from C₁~C₆ alkyl, deuterated C₁~C₆ alkyl or C₃~C₆ cycloalkyl, wherein the C₁~C₆ alkyl or C₃~C₆ cycloalkyl is optionally substituted with one or more halogens;
or, R₁ is selected from C₁~C₆ alkyl, two hydrogen atoms on the same carbon atom of the C₁~C₆ alkyl are simultaneously substituted with -(CH₂)ⱼ-, thereby forming C₃~C₆ cycloalkyl, wherein j is 1, 2, 3, 4 or 5;
R₂, R₃ are each independently selected from hydrogen, C₁~C₆ alkyl, deuterated C₁~C₆ alkyl, halogenated C₁~C₆ alkyl or C₃~C₆ cycloalkyl,
X, Y are each independently selected from O atom or absent, provided that at least one of X and Y is O;
m, n are each independently selected from 0, 1, 2 or 3.

In one embodiment, R₁ is selected from C₁~C₆ alkyl or deuterated C₁~C₆ alkyl;
preferably, R₁ is selected from -CH₂-, -CD₂-, -(CH₂)₂- or -CH(CH₃)-; or,
R₁ is selected from C₁~C₃ alkyl, two hydrogen atoms on the same carbon atom of the C₁~C₃ alkyl are simultaneously substituted with -(CH₂)ⱼ-, thereby forming cyclopropyl, cyclobutyl, cyclopentyl or cyclohexyl;
preferably, R₁ is selected from

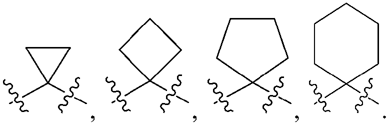

In one embodiment, R₂ is selected from C₁~C₆ alkyl or deuterated C₁~C₆ alkyl;
preferably, R₂ is selected from methyl, ethyl or deuterated methyl.

In one embodiment, R₃ is selected from hydrogen or C₁~C₆ alkyl.
m, n are each independently selected from 0, 1 or 2;
preferably, 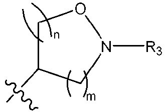 is selected from

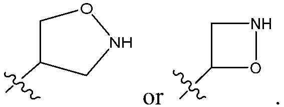

In one embodiment, X is O, Y is absent, or Y is O, X is absent.

In one embodiment, the compound of formula (I) is the compound represented by formula (IIa): 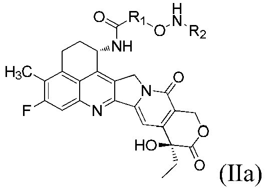
in formula (IIa), R₁, R₂ are each as defined in the compound of formula (I);
preferably, R₁ is selected from -CH₂-, -CD₂-, -(CH₂)₂-, -CH(CH₃)- or 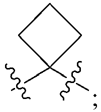
preferably, R₂ is selected from methyl, ethyl or deuterated methyl.

In one embodiment, the compound of formula (I) is the compound represented by formula (IIb): 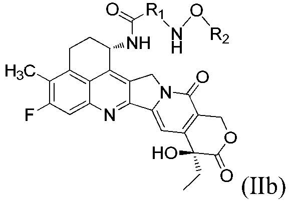 in formula (IIb), R₁, R₂ are each as defined in the compound of formula (I), preferably, R₁ is selected from -CH₂-, -CD₂-, -(CH₂)₂-, -CH(CH₃)- or 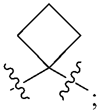 preferably, R₂ is selected from methyl, ethyl or deuterated methyl.

In one embodiment, the compound of formula (I) is the compound represented by formulas (D1)~(D5):

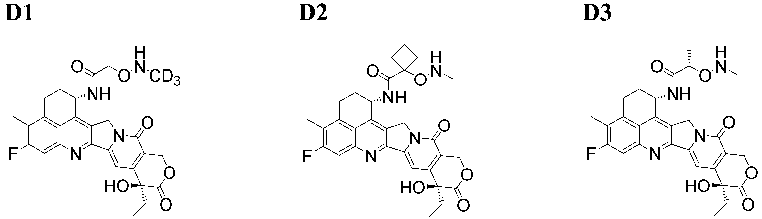

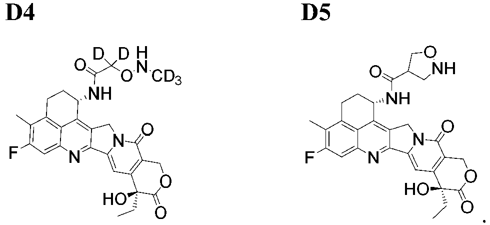

Another aspect of the present disclosure provides a method for preparing the above-mentioned compound, comprising: reacting the -2'(S)-amino- group of the 7,9-fused cyclohexane moiety in the exatecan structure with the corresponding hydroxylamine-containing substituted compound to prepare exatecan hydroxylamine derivatives;
wherein,
the amino group in the hydroxylamine-containing substituted compound is subjected to amino protection, wherein the amino protection is carried out by substituting the hydrogen atom on the amino group with an amino protecting group; preferably, the amino protecting group is tertbutoxycarbonyl (Boc);
the acyl group in the hydroxylamine-substituted compound is selected from any one of hydroxylamine-substituted acetyl, hydroxylamine-substituted propionyl, hydroxylamine-substituted cyclobutanecarbonyl, hydroxylamine-substituted isoxazolidine-4'-carbonyl, hydroxylamine-substituted deuterated acetyl.

In one embodiment, the hydroxylamine-containing substituted compound is selected from any one of

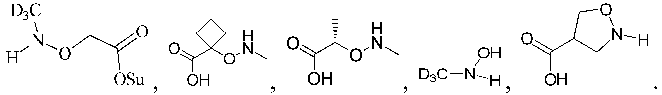

In one embodiment, the preparation method comprises forming an amide between the -2'(S)-amino group of the 7,9-fused cyclohexane moiety of exatecan and a hydroxylamine-substituted acetyl group.

In one embodiment, the preparation method comprises forming an amide between the -2'(S)-amino group of the 7,9-fused cyclohexane moiety of exatecan and a hydroxylamine-substituted propionyl group.

In one embodiment, the preparation method comprises forming an amide between the -2'(S)-amino group of the 7,9-fused cyclohexane moiety of exatecan and a hydroxylamine-substituted cyclobutanecarbonyl group.

In one embodiment, the preparation method comprises forming an amide between the -2'(S)-amino group of the 7,9-fused cyclohexane moiety of exatecan and a hydroxylamine-substituted isoxazolidine-4'-carbonyl group.

In one embodiment, the preparation method comprises forming an amide between the -2'(S)-amino group of the 7,9-fused cyclohexane moiety of exatecan and a hydroxylamine-substituted deuterated acetyl group.

A further aspect of the present disclosure provides an antibody-drug conjugate, comprising a small molecule drug, a linker, and an antibody, wherein, the small molecule drug comprises the above-mentioned compound, or pharmaceutically acceptable salt, stereoisomer, or prodrug.

A further aspect of the present disclosure provides a pharmaceutical composition comprising the above-mentioned compound, or pharmaceutically acceptable salt, stereoisomer, or prodrug thereof, and a pharmaceutically acceptable excipient.

A further aspect of the present disclosure provides a use of the above-mentioned compound, or pharmaceutically acceptable salt, stereoisomer, or prodrug thereof, the antibody-drug conjugate, or the pharmaceutical composition in the manufacture of a medicament for treating a cancer.

A further aspect of the present disclosure provides a method for treating a cancer, comprising the step of administering to a patient in need thereof a therapeutically effective amount of the above-mentioned compound, or pharmaceutically acceptable salt, stereoisomer, or prodrug thereof, the antibody-drug conjugate, or the pharmaceutical composition.

The above-mentioned compound, or pharmaceutically acceptable salt, stereoisomer, or prodrug thereof, can be used alone, in combination, or in the form of an antibody-drug conjugate for the treatment of cancer.

Preferably, the cancer comprises any one or more of esophageal adenocarcinoma, gastric cancer, esophageal cancer, lung cancer, lung squamous cell carcinoma, breast cancer, breast adenocarcinoma and bladder cancer.

### DETAILED DESCRIPTION OF THE EMBODIMENTS

### I. Definition

In the present disclosure, unless otherwise specified, scientific and technical terms used herein have the meanings commonly understood by those skilled in the art. Also, the relevant terms and laboratory procedures used herein are terms and conventional procedures widely used in the corresponding fields. Meanwhile, for a better understanding of the present disclosure, definitions and explanations of related terms are provided below.

As used herein and unless otherwise specified, the terms "comprising", "including", "having", "containing", including grammatical equivalents thereof, should generally be understood to be open-ended and non-limiting, for example, not excluding other unrecited elements or steps.

The compound of the present disclosure can be asymmetric, for example, having one or more stereoisomers. Unless otherwise specified, all stereoisomers are included, for example, enantiomers and diastereomers. The stereoisomers include geometric isomers (such as cis and trans structures) and optical isomers (such as enantiomers), therapeutic substances consisting of monomers, racemates, racemic mixtures and pharmaceutically acceptable salts thereof. The compound of the present disclosure containing asymmetric carbon atoms can be isolated in optically active pure form or racemic form. Optically active pure forms can be resolved from racemic mixtures or synthesized by using chiral starting materials or chiral reagents. Racemates, diastereomers, enantiomers are all included within the scope of the present disclosure.

The compound of the present disclosure also includes tautomeric forms. Tautomeric forms arise from the exchange of one single bond with an adjacent double bond and together with the transfer of one proton.

As used herein, "pharmaceutically acceptable salt" refers to the salt of the corresponding amine compound with an inorganic or organic acid, or the salt of the corresponding carboxylic acid compound with an alkali metal or alkaline earth metal, or with an organic amine. Wherein, inorganic acids include but are not limited to hydrochloric acid, hydrobromic acid, hydroiodic acid, sulfuric acid, phosphoric acid and so on; organic acids include but are not limited to acetic acid, propionic acid, butyric acid, benzoic acid, methanesulfonic acid, toluenesulfonic acid, p-toluenesulfonic acid, oxalic acid, succinic acid, lactic acid, citric acid, succinic acid, gluconic acid, maleic acid, fumaric acid, tartaric acid and so on; alkali metal or alkaline earth metal salts include but are not limited to sodium, potassium, calcium, magnesium salts and so on; organic amine salts include, but are not limited to, salts composed of ammonia, methylamine, ethylamine, propylamine, isopropylamine, dimethylamine, diethylamine, trimethylamine, triethylamine, tert-butylamine, ethylenediamine, ethanolamine, diethanolamine, triethanolamine, morpholine, piperidine, piperazine, amino acids, etc.

As used herein, "prodrug" refers to the form in which a compound is metabolized or simply chemically altered in the body of a patient after the compound has been introduced into the human body in a suitable mode of administration to the compound contained in general formula 1 of the present invention, as well as the corresponding salt. Prodrugs of the compounds include, but are not limited to, various carboxylate esters, carbonate esters, phosphate esters, sulfate esters, sulfonate esters, amino acid esters, gluconate esters, as well as various amides, acetals, hemiacetals, carbamide esters, etc.

The numerical range as used herein refers to each integer within the given range. For example,"C₁~C₆" refers to the group that can have 1 carbon atom, 2 carbon atoms, 3 carbon atoms, 4 carbon atoms, 5 carbon atoms or 6 carbon atoms; "C₃~C₆" refers to the group that can have 3 carbon atoms, 4 carbon atoms, 5 carbon atoms or 6 carbon atoms.

When any variable (such as Rn) appears more than once in the composition or structure of a compound, the definition thereof is independent in each case. Therefore, for example, if a group is substituted with 1-5 R's, then the group can optionally be substituted with up to 5 R's, and each R has independent options in every case. In addition, combinations of substituents and/or variants thereof are only allowed if such combinations result in the formation of stable compounds.

The term "alkyl" refers to saturated aliphatic hydrocarbon groups, which is a straight or branched chain group containing 1 to 20 carbon atoms, preferably the alkyl containing 1 to 8 carbon atoms, more preferably 1 to 6 carbon atoms, and most preferably 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 2,2-diethylhexyl, 2,2-diethylhexyl, and various branched isomers thereof, etc., more preferably, a lower alkyl containing 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Alkyl can be substituted or unsubstituted, and when substituted, substituents can be present at any available point of attachment, the substituents preferably being one or more of the following groups independently selected from alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl, or carboxylate group, with methyl, ethyl, isopropyl, tert-butyl, halogenated alkyl, deuterated alkyl, alkoxy-substituted alkyl, and hydroxy-substituted alkyl being preferred for the present disclosure.

The hydrogen atoms of the present disclosure can be substituted by the isotope deuterium thereof.

The term "substituted" refers to one or more hydrogen atoms in a group, preferably up to 5, and more preferably 1-3 hydrogen atoms, being independently substituted with the corresponding number of substituents. It goes without saying that the substituents are only in their possible chemical positions, and those skilled in the art can determine (through experiment or theory) which substitutions are possible or impossible without undue effort. For example, combinations such as an amino or hydroxy having a free hydrogen with a carbon atom having an unsaturated (e.g., olefinic) bond may be unstable. 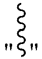 refers to the chemical bond junction.

Medicament or pharmaceutical composition

The drugs or drug compositions of the present disclosure can be administered orally, topically, parenterally, or mucosally (e.g., sublingually, by inhalation, or rectally) in dose unit formulations comprising conventional non-toxic pharmaceutically acceptable carriers. An oral route is generally preferred. The active agents can be administered orally in the form of capsules, tablets, etc. (see Remington: The Science and Practice of Pharmacy, 20th Edition).

For oral administration in the form of tablets or capsules, the active pharmaceutical ingredients can be combined with non-toxic, pharmaceutically acceptable excipients such as binders (for example, pregelatinized corn starch, polyvinyl pyrrolidone, or hydroxypropyl methylcellulose); fillers (for example, lactose, sucrose, glucose, mannitol, sorbitol, and other reducing and non-reducing sugars, microcrystalline cellulose, calcium sulfate, or calcium hydrogen phosphate); lubricants (for example, magnesium stearate, talc, or silica, stearic acid, sodium stearyl fumarate, glyceryl behenate, calcium stearate, etc.); disintegrants (for example, potato starch or sodium starch glycolate); or wetting agents (for example, sodium lauryl sulfate), colorants and flavorings, gelatin, sweeteners, natural and synthetic gums (such as acacia gum, tragacanth gum, or alginates), buffering salts, carboxylmethyl cellulose, polyethylene glycol, waxes, etc. For oral administration in liquid form, the pharmaceutical ingredients can be combined with non-toxic, pharmaceutically acceptable inert carriers (for example, ethanol, glycerin, water), anti-sedimentation agents (for example, sorbitol syrup, cellulose derivatives, or hydrogenated edible fats), emulsifiers (for example, lecithin or acacia gum), non-aqueous carriers (for example, almond oil, oil esters, ethanol, or fractionated vegetable oils), preservatives (for example, methyl p-hydroxybenzoate or propyl p-hydroxybenzoate or sorbic acid), etc. Stabilizers such as antioxidants (BHA, BHT, propyl gallate, sodium ascorbate, citric acid) can also be added to stabilize the dosage form.

Tablets containing the active compound can be coated using methods well-known in the field. The compositions of the present disclosure, which include the compound represented by formula I as the active compound, can also be introduced into small beads, microspheres, or microcapsules, for example, constructed with polyglycolic acid/lactic acid (PGLA). Liquid formulations for oral administration can take the forms of e.g. solution, syrup, emulsion, or suspension, or they can be presented as dry products that are reconstituted with water or other suitable excipients before use. Formulations for oral administration can be suitably formulated to provide controlled or delayed release of the active compound.

The term "treatment" includes inhibiting, alleviating, preventing, or eliminating one or more symptoms or side effects associated with the disease, condition, or disorder being treated.

The term "inhibition" is used in relation to controls. Those skilled in the art will readily determine the appropriate control for each experiment. For example, a reduced response in subjects or cells treated with the compound is compared to the response in subjects or cells not treated with the compound.

The term "pharmaceutical composition" refers to a composition that includes the compound or pharmaceutically acceptable salt thereof of the present disclosure, as well as at least one pharmaceutically acceptable component selected from the following, depending on the administration route and the nature of dosage form, including but not limited to: carriers, diluents, adjuvants, excipients, preservatives, fillers, disintegrants, wetting agents, emulsifiers, suspension agents, sweeteners, flavoring agents, fragrances, antimicrobials, antifungals, lubricants, dispersing agents, temperature-sensitive materials, temperature regulators, adhesives, stabilizers, suspending agents, etc.

The term "effective amount" or "therapeutically effective amount" refers to a sufficient amount of a drug or medication that is non-toxic but can achieve the desired effect. In embodiments of the present disclosure, when treating a patient in accordance with the present disclosure, the amount of drugs administered depends on many factors, such as the specific dosing regimen, the type of disease or condition and the severity thereof, the uniqueness (for example, body weight) of the treated person or host to be treated, but the amount of drug administered depends on specific surrounding circumstances, including, for example, the specific drug that has been employed, the route of administration, the treated condition, and the treated person or host to be treated, the administered dose may be routinely determined by methods known in the art. Generally, regarding the dosage used for adult therapy, the administered dose is typically within the range of 0.02-5000mg/day, for example, the range of about 1-1500mg/day. The required dose can be conveniently administered as a single dose, or as doses given simultaneously (or in a short period of time) or in divided doses at appropriate intervals, such as two, three, four doses per day or more. It can be understood by those skilled in the art that, although the above dose ranges are provided, the specific effective amount can be appropriately adjusted based on the patient's condition and in conjunction with a physician's diagnosis.

The term "antibody-drug conjugate (ADC)" refers to a biologically active small molecule drug linked to a monoclonal antibody via a chemical linker. The monoclonal antibody serves as a carrier to target and deliver the small molecule drug to specific target cells.

As used herein, the terms "reduce", "inhibit", "alleviate", or "decrease" are used in relation to controls. Those skilled in the art will easily determine the appropriate control for each experiment. For example, a reduced response in subjects or cells treated with the compound is compared to the response in subjects or cells not treated with the compound.

Unless otherwise specified, the raw materials and equipment used in the specific embodiments of the present disclosure are known products that can be obtained by purchasing commercial products.

### Cells used:

OE33: human esophageal cancer cells;
SK-BR-3: human breast cancer cells;
NCI-N87: human gastric cancer cells;
NCI-H1975: human lung cancer cells;
MDA-MB-231: human breast cancer cells;
NCI-H226: human lung squamous cell carcinoma cells;
TE12: human esophageal cancer cells.

### II. Examples

In order to make the objectives, technical solutions, and advantages of the present disclosure clearer, the present disclosure will be described in further detail below. The described Examples shall not be construed as limiting the present disclosure, and all other Examples obtained by those of ordinary skill in the art without creative work shall fall within the protection scope of the present disclosure.

Before describing the Examples of the present disclosure in further detail, the nouns and terms involved in the Examples of the present disclosure are explained, and the nouns and terms involved in the Examples of the present disclosure are applicable to the following explanations.

The raw materials and equipment used in the specific embodiments of the present disclosure are all known products and are obtained by purchasing commercially available products.

### Example 1: N-(2'-O-(N-trideuteriomethylamino)oxyacetyl)exatecan (D1)

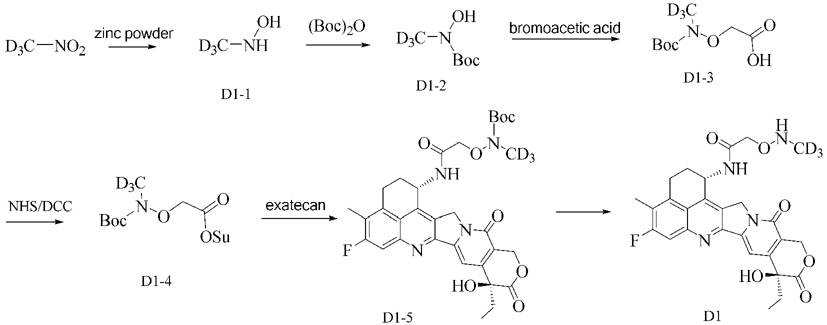

To a 100 mL flask charged with 35 mL of H₂O, CD₃NO₂ (2.24 g, 2.0 mL, 35 mmol) and NH₄Cl (1.50 g, 28 mmol) were added. The mixture was stirred in an ice-water bath, and zinc powder (5.7 g, 87.5 mmol) was added portionwise over 1 hour. After stirring for an additional 2 hours, the mixture was filtered and washed with water. The filtrate was acidified with concentrated HCl (aq) to pH=3 and evaporated to dryness. CD₃NHOH was recrystallized from EtOH/Et₂O to afford **D1-1,** which was used without further purification.

At 25°C, **D1-1** (4.2 g, 50.29 mmol) was dissolved in THF (100 mL) in a 250 mL round-bottom flask. Boc anhydride (11.03 g, 50.29 mmol, 11.59 mL) and K₂CO₃ (6.95 g, 50.29 mmol) were added successively. The mixture was magnetically stirred to dissolve most of the solids, and the reaction was carried out at 25°C for 24 hours under nitrogen protection. The reaction mixture was extracted three times by adding ethyl acetate. The combined organic layer was washed with saturated brine, dried over anhydrous magnesium sulfate, and concentrated to afford **D1-2** (5.36 g, 36.17 mmol, 71.93% yield) as a pale yellow liquid.

At 25°C, **D1-2** (1 g, 6.61 mmol) was dissolved in H₂O (15 mL) in a 250 mL round-bottom flask. Bromoacetic acid (1.16 g, 8.27 mmol) and NaOH (264.53 mg, 6.61 mmol) were added successively. The mixture was stirred to dissolve most of the solids, and the mixture was reacted at 25°C for 24 hours under nitrogen protection. The reaction mixture was extracted three times by adding ethyl acetate, washed with saturated brine, dried over anhydrous magnesium sulfate, and concentrated to afford **D1-3** (1.36 g, 5.52 mmol, 83.54% yield).

At 0°C, **D1-3** (500 mg, 2.44 mmol) was dissolved in DCM (10 mL) in a 250 mL round-bottom flask. EDCI (467.1 mg, 2.44 mmol) and NHS (280.1 mg, 2.44 mmol) were added successively. The mixture was stirred to dissolve most of the solids, and the mixture was reacted at 25°C for 3 hours under nitrogen protection to afford **D1-4.**

At 25°C, exatecan (100 mg, 188.13 µmol) was dissolved in DMF (1 mL) in a 25 mL round-bottom flask. TEA (30.5 mg, 301.00 µmol) and **D1-4** (91 mg, 301.00 µmol) were added successively. The mixture was stirred to dissolve most of the solids, and the mixture was reacted at 25°C for 2 hours under nitrogen protection. Silica gel was added to the reaction mixture, and the mixture was purified by silica gel column chromatography (DCM:MeOH = 97:3), concentrated to afford **D1-5** (85.3mg, 72.8%) as a yellow solid; LCMS: [M+1]⁺ 626.21 (calculated value: 625.67).

**D1-5** (53 mg, 84.57 µmol) was dissolved in EA (0.5 mL). 4 M HCl/EA (0.5 mL) was added. The mixture was stirred to dissolve most of the solids. The mixture was reacted at 25°C for 2 hours under nitrogen protection, concentrated to afford **D1** (30.2 mg, 45.80 µmol, yield 54.16%) as a yellow solid; ¹H NMR (600 MHz, DMSO-d₆) δ 8.53 (d, *J*=8.8 Hz, 1H), 7.81 (d, *J*=10.9 Hz, 1H), 7.31 (s, 1H), 5.66-5.57 (m, 1H), 5.42 (s, 2H), 5.26 (s, 1H), 4.28 (s, 2H), 3.18 (t, *J*=6.6 Hz, 2H), 2.41 (s, 3H), 2.18 (dt, *J*=36.9, 7.2 Hz, 2H), 1.86 (ddd, *J*=21.4, 14.2, 7.0 Hz, 2H), 0.87 (t, *J*=7.3 Hz, 3H).

### Example 2: N-(1'-O-(N-methylamino)oxycyclobutane-1'-carbonyl)exatecan (D2)

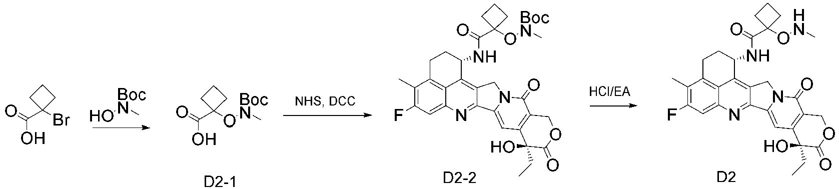

In a 10 mL vial, NaOH (555.46 mg, 13.89 mmol), H₂O (1 mL), and tert-butyl N-hydroxy-N-methylcarbamate (82.31 mg, 555.50 µmol) were added. Finally, a 0.5 mL aqueous solution of 1-bromocyclobutanecarboxylic acid (100 mg, 555.50 µmol) was added dropwise to the reaction vial. The mixture was stirred at 25°C for 1 h, extracted with DCM, and concentrated to afford **D2-1** (100 mg, yield 67.98%, 93% HPLC); LCMS: [M+1]⁺ 245.83 (calculated value: 245.13).

Exatecan (159.50 mg, 365.44 µmol) was dissolved in DMF (1 mL). **D2-1** (100 mg, 406.04 µmol), EDCI (77.84 mg, 406.04 µmol), NHS (46.73 mg, 406.04 µmol), and DCC (83.78 mg, 406.04 µmol) were added successively. The mixture was stirred at 25 °C for 2 h and purified by silica gel column chromatography to afford **D2-2** (130 mg, 168.45 µmol, yield 41.48%, HPLC 94 %); LCMS: [M+1]⁺ 663.27 (calculated value: 662.28).

**D2-2** (100 mg, 150.67 µmol) was dissolved in DCM (5 mL). TFA (34.36 mg, 301.34 µmol, 23.06 µL) was added, and the mixture was stirred at room temperature for 1 h. DCM was concentrated, and purification was performed using a C18 Spherical 20-35 µm 100 Å 40 g reversed-phase column. The mobile phases consisted of acetonitrile and 0.05% TFA aqueous solution, designated as mobile phase B2 and A2, respectively. Purification was carried out by preparative HPLC with detection at 254 nm and 214 nm to afford **D2.** (50 mg, 86.94 µmol, yield 57.70%, HPLC 98 %); ¹H NMR (600 MHz, DMSO-d₆) δ 8.29 (d, J=8.5 Hz, 1H), 7.76 (d, J=10.7 Hz, 1H), 7.31 (s, 1H), 5.57 (q, J=7.3, 6.9 Hz, 1H), 5.41 (s, 2H), 5.15 (s, 1H), 5.11 (s, 1H), 4.05 (s, 2H), 3.23-3.07 (m, 2H), 2.71 (s, 3H), 2.38 (s, 3H), 2.16 (q, J=6.5 Hz, 2H), 1.86 (dtt, J=21.5, 14.4, 6.6 Hz, 2H), 1.24 (tp, J=12.7, 7.1, 6.4 Hz, 2H), 0.88 (q, J=7.4, 6.4 Hz, 6H); LCMS: [M+1]⁺ 563.26 (calculated value: 562.22).

### Example 3: N-(2'-methyl-2'-O-(N-methylamino)oxyacetyl)exatecan (D3)

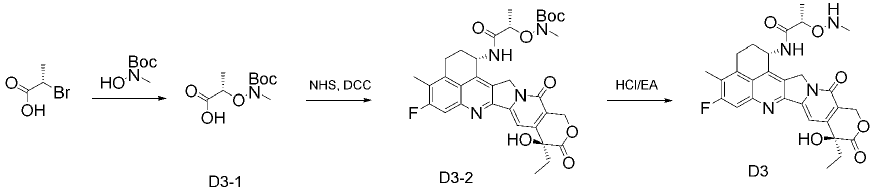

To a reaction vial, a solution of NaOH (64.94 mg, 1.62 mmol) in H₂O (1 mL) was added. Then (2S)-2-bromopropanoic acid (100 mg, 649.43 µmol) and tert-butyl N-hydroxy-N-methylcarbamate (96.23 mg, 649.43 µmol) were added successively. The mixture was stirred at room temperature for 2 h, extracted with DCM, and concentrated to afford the product **D3-1** (80 mg, 290.59 µmol, yield 44.75%, HPLC 94%) LCMS: [M+1]⁺ 220.23 (calculated value: 219.24).

Exatecan (142.68 mg, 326.91 µmol) was dissolved in DMF (2.04 mL). TEA (110.27 mg, 1.09 mmol, 151.88 µL), **D3-1** (80 mg, 363.24 µmol), EDCI (69.63 mg, 363.24 µmol), and NHS (41.80 mg, 363.24 µmol) were added successively. The mixture was stirred at 25 °C for 1 h, extracted with DCM, and purified by silica gel column chromatography to afford **D3-2** (130 mg, 177.36 µmol, yield 48.83%, HPLC 97 %); LCMS: [M+1]⁺ 637.54 (calculated value: 636.68).

**D3-2** (90 mg, 141.14 µmol) was dissolved in DCM (5 mL). TFA (2.98 g, 26.14 mmol, 2 mL) was added, and the mixture was stirred at 25 °C for 1 h. Purification was performed using a C18 Spherical 20-35 µm 100 Å 40 g reversed-phase column. The mobile phases consisted of acetonitrile and 0.05% TFA aqueous solution, designated as mobile phase B2 and A2, respectively. Purification was carried out by preparative HPLC with detection at 254 nm and 214 nm to afford the product **D3** (40 mg, 72.92 µmol, yield 51.67%, HPLC 98 %); ¹H NMR (600 MHz, DMSO-d₆) δ 8.49 (d, J=8.7 Hz, 1H), 7.74 (t, J=9.3 Hz, 1H), 7.29 (s, 1H), 6.50 (s, 1H), 5.57 (q, J=6.6, 6.2 Hz, 1H), 5.41 (s, 2H), 5.24 (s, 0H), 5.23-5.14 (m, 1H), 5.12 (s, 0H), 4.39-4.35 (m, 0H), 4.32 (q, J=6.8 Hz, 1H), 3.18 (td, J=15.7, 15.0, 6.8 Hz, 1H), 3.12 (s, 0H), 2.50 (s, 2H), 2.37 (s, 2H), 2.34 (d, J=20.3 Hz, 1H), 2.16 (q, J=6.3 Hz, 2H), 2.13 (s, 0H), 1.87 (td, J=13.9, 7.2 Hz, 2H), 1.82 (d, J=7.0 Hz, 0H), 1.42 (t, J=7.4 Hz, 1H), 1.34 (d, J=6.8 Hz, 2H), 0.86 (t, J=7.3 Hz, 3H); LCMS: [M+1]⁺ 537.80 (calculated value: 536.56).

### Example 4: N-(2',2'-dideuterio-2'-O-(N-trideuteriomethylamino)oxyacetyl)exatecan (D4)

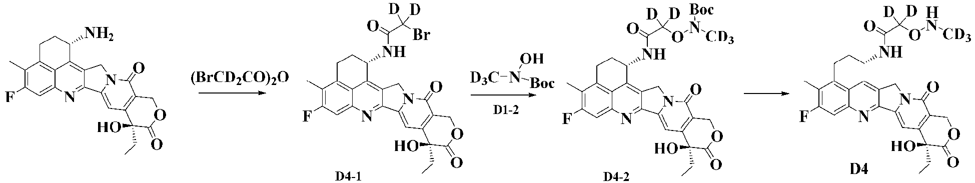

Under nitrogen protection at room temperature, exatecan (4 g, 7.77 mmol) and DMF (10 mL) were added to a 50 mL round-bottom flask and stirred to dissolve. 2-Bromo-2,2-dideuterioacetic anhydride (2.06 g, 7.77 mmol) and DIEA (1.00 g, 7.77 mmol) were added successively and stirred to dissolve. The mixture was reacted at room temperature for 24 hours. The mixture was extracted three times by adding ethyl acetate, washed with saturated brine, dried over anhydrous magnesium sulfate, and concentrated to afford **D4-1** (4.05 g, yield 88.47%) as a pale yellow solid; LCMS: [M+1]⁺ 559.37 (calculated value: 558.40).

Under nitrogen protection at room temperature, **D4-1** (2 g, 3.58 mmol) was dissolved in DMF (10 mL). **D1-2** (0.753 g, 5.01 mmol) and DBU (0.818 g, 5.37 mmol) were added successively. The mixture was magnetically stirred to dissolve most of the solids, and the mixture was stirred at room temperature overnight. The mixture was extracted three times by adding ethyl acetate, washed with saturated brine, dried over anhydrous magnesium sulfate, and concentrated to afford **D4-2** (1.05 g, yield 47% yield) as a pale yellow solid; LCMS: [M+1]⁺ 628.58 (calculated value: 527.68).

Under nitrogen protection at 25°C, **D4-2** (0.7 g, 1.12 mmol) was dissolved in DCM (10 mL). TFA (1 mL) was added, and the mixture was stirred for 0.5 h, then concentrated and purified to afford **D4** (0.536 g, yield 91% ) as a pale yellow solid; ¹H NMR (500 MHz, DMSO-*d*₆) δ 8.64 (dd, *J*=8.9, 5.5 Hz, 1H), 7.78 (dd, *J*=15.1*,* 10.8 Hz, 1H), 7.30 (d, *J*=7.8 Hz, 1H), 6.55 (s, 1H), 5.61 (dt, *J*=9.2, 4.7 Hz, 1H), 5.42 (d, *J*=4.0 Hz, 2H), 5.24 (d, *J*=8.5 Hz, 2H), 3.18 (t, *J=*6.4 Hz, 3H), 2.39 (d, *J*=7.9 Hz, 3H), 2.28-2.10 (m, 2H), 1.86 (dp, *J*=21.4, 7.1 Hz, 2H), 0.87 (t, *J*=7.4 Hz, 3H); LCMS: [M+1]⁺ 528.46(calculated value: 527.56).

### Example 5: N-(isoxazolidine-4'-carbonyl)exatecan (D5)

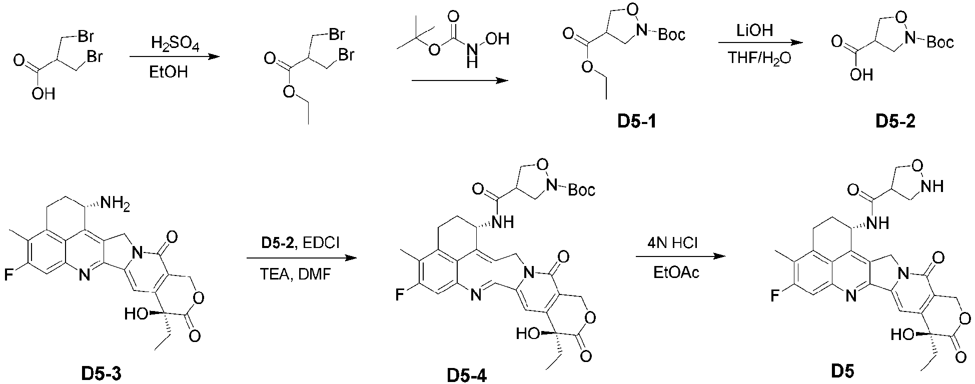

Concentrated sulfuric acid (0.5 mL) was added to a solution of 3-bromo-(2-bromomethyl)propanoic acid (3 g, 12.15 mmol) in ethanol (30 mL). The mixture was stirred and heated to reflux for 18 h. The mixture was concentrated, extracted with methyl tert-butyl ether, washed with brine, and concentrated to afford ethyl 3-bromo-(2-bromomethyl)propanoate as an oily liquid, which was used in the next step without further purification.

To a reaction vial, N-Boc hydroxylamine (3 g, 22.36 mmol), DMF (10 mL), and 60% NaH (1.79 g, 44.72 mmol) were added. The mixture was stirred at 0 °C for 10 min, and ethyl 3-bromo-(2-bromomethyl)propanoate (6.15 g, 22.36 mmol) was added. The mixture was reacted at room temperature for 3 h, poured into ice water, extracted with methyl tert-butyl ether, and purified by silica gel column chromatography to afford **D5-1** (1.16 g, yield 21%). **D5-1** was dissolved in 1:1 THF/H₂O (10 mL), and LiOH (252.82 mg, 10.56 mmol) was added. The mixture was stirred at room temperature for 3 h, concentrated, acidified with 1 N aqueous HCl solution, extracted with DCM, and concentrated to afford **D5-2** (0.9 g, yield 78 %).

To a reaction vial, **D5-3** (1.46 g, 3.35 mmol), **D5-2** (0.73 g, 3.35 mmol), DMF (10 mL), EDCI (961.90 mg, 5.02 mmol), and triethylamine (507.74 mg, 5.02 mmol, 699.37 µL) were added. The mixture was stirred at room temperature for 3 h, concentrated, and purified by silica gel column chromatography to afford **D5-4** (700 mg, yield 19%) as a white solid. The obtained **D5-4** was dissolved in 4 M HCl in EtOAc (5 mL), stirred at room temperature for 3 h, and concentrated to afford **D5** (170 mg, yield 82 %) as a white solid; ¹H NMR (500 MHz, DMSO-*d*₆) δ 7.81 (dd, *J=*11.1*,* 4.2 Hz, 1H), 7.31 (d, *J*=3.5 Hz, 1H), 5.82 (dd, *J*=19.1, 13.1 Hz, 1H), 5.49-5.31 (m, 4H), 5.16 (d, *J*=14.4 Hz, 2H), 3.74 (dt, *J=12.5,* 6.2 Hz, 1H), 3.69-3.31 (m, 5H), 3.23 (ddd, *J*=18.3, 12.8, 5.5 Hz, 1H), 2.85 (d, *J*=14.7 Hz, 1H), 2.37 (d, *J*=5.2 Hz, 4H), 2.22 (t, *J*=13.1 Hz, 1H), 1.87 (tt, *J*=14.4, 6.5 Hz, 3H), 0.87 (t, *J*=7.2 Hz, 3H); ¹³C NMR (126 MHz, DMSO-*d*₆) δ 172.85, 171.98, 171.86, 163.20, 161.21, 157.13, 152.91, 150.43, 148.34, 148.23, 145.49, 135.92, 133.07, 129.22, 129.11, 125.44, 125.29, 121.25, 121.14, 120.00, 119.62, 110.75, 110.57, 97.44, 72.81, 65.80, 53.04, 52.82, 50.93, 49.69, 49.25, 44.71, 44.62, 40.47, 40.30, 40.14, 39.97, 39.80, 39.63, 39.47, 38.00, 37.83, 30.92, 22.96, 22.59, 21.74, 11.54, 8.29. LCMS [M+H]⁺ 535.20 (calculated value 534.19).

### Effect Example 1: Tumor cell growth inhibitory activity

Tumor cells OE33, SK-BR-3, NCI-N87, NCI-H1975, MDA-MB-231, NCI-H226, and TE12 were cultured separately in RPMI1640 (Cellmax) supplemented with 10% fetal bovine serum (Cellmax). Tumor cells in the exponential growth phase were diluted to a concentration of 1×10⁵ cells/mL with culture medium and added to 96-well cell culture plates at 100 µL per well, then returned and incubated overnight in a 37°C incubator with 5% CO₂. On the following day, the compounds were diluted to concentrations of 10000 nM, 2000 nM, 400 nM, 80 nM, 16 nM, 3.2 nM, 0.64 nM, and 0.13 nM with culture medium, and the diluted compounds were added to the 96-well cell culture plates at 2 µL per well, with three replicate wells for each concentration. Negative control and blank control groups without addition of compounds were added with 2 µL of the dilution liquid per well. After the addition of the compounds, the plates were returned to the 37°C incubator with 5% CO₂ to continue the incubation for 72 h. After the incubation, the cell culture plates were removed, and the culture medium in the plates was aspirated using a pipette. Then, 100 µL of culture medium containing 10% CCK-8 was added to each well, and the plates were incubated at 37°C for 3 h. After the incubation, the plates were removed, kept in the dark, and placed in an ELISA plate. Absorbance was measured at 630 nm as the reference wavelength and 450 nm as the test wavelength. Based on the absorbance values, the IC₅₀ values (Table 1) were calculated using the four-parameter logistic regression method in GraphPad.

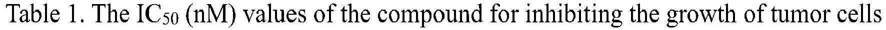

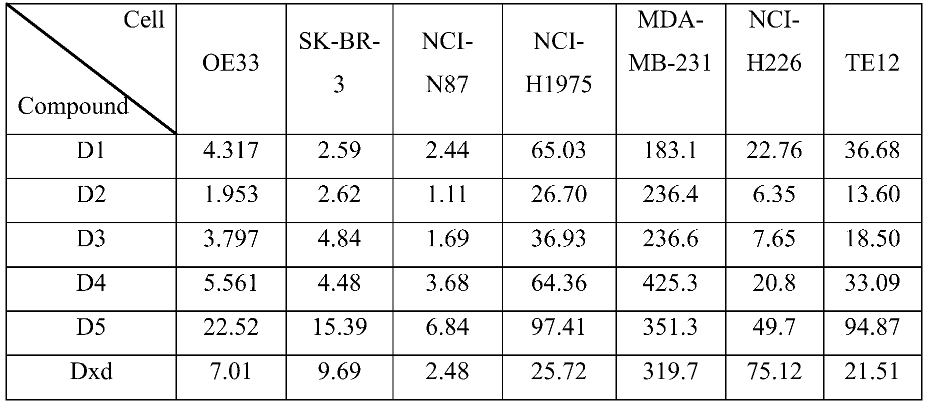

"Dxd" was the positive control drug, and its structural formula is "

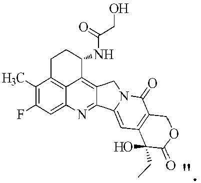

As can be seen from the results in the table, the exemplary compounds in the Examples of the present disclosure exhibit good inhibitory effects on the growth of tumor cells, and their IC₅₀ values for growth inhibition are all below 500 nM, or below 100 nM or 50 nM, and even below 10 nM.

The foregoing descriptions of specific exemplary embodiments of the present disclosure have been presented for purposes of illustration and example. These descriptions are not intended to limit the present disclosure to the precise forms disclosed, and obviously many modifications and variations are possible in light of the above-mentioned teachings. The exemplary embodiments were chosen and described in order to explain the particular principles of the present disclosure and its practical application, to thereby enable those skilled in the art to make and use various exemplary embodiments of the present disclosure and various alternatives and modifications. It is intended that the scope of the present disclosure be defined by the claims and their equivalents.

## Claims

1. A compound of formula (I), or a pharmaceutically acceptable salt, a stereoisomer, or a prodrug thereof: 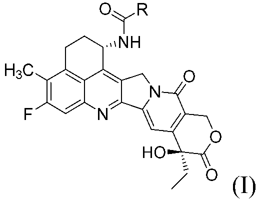
in the Formula, R is selected from 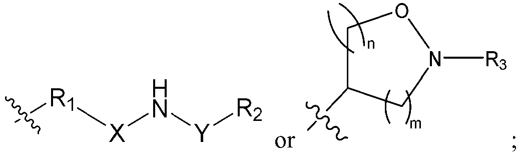
wherein, R₁ is selected from C₁~C₆ alkyl, deuterated C₁~C₆ alkyl or C₃~C₆ cycloalkyl, wherein the C₁~C₆ alkyl or C₃~C₆ cycloalkyl is optionally substituted with one or more halogens;
or, R₁ is selected from C₁~C₆ alkyl, two hydrogen atoms on the same carbon atom of the C₁~C₆ alkyl are simultaneously substituted with -(CH₂)ⱼ-, thereby forming C₃~C₆ cycloalkyl, wherein j is 1, 2, 3, 4 or 5;
R₂, R₃ are each independently selected from hydrogen, C₁~C₆ alkyl, deuterated C₁~C₆ alkyl, halogenated C₁~C₆ alkyl or C₃~C₆ cycloalkyl,
X, Y are each independently selected from O atom or absent, provided that at least one of X and Y is O;
m, n are each independently selected from 0, 1, 2 or 3.

2. The compound according to claim 1, wherein, R₁ is selected from C₁~C₆ alkyl or deuterated C₁~C₆ alkyl;
preferably, R₁ is selected from -CH₂-, -CD₂-, -(CH₂)₂- or -CH(CH₃)-; or,
R₁ is selected from C₁~C₃ alkyl, two hydrogen atoms on the same carbon atom of the C₁~C₃ alkyl are simultaneously substituted with -(CH₂)ⱼ-, thereby forming cyclopropyl, cyclobutyl, cyclopentyl or cyclohexyl;
preferably, R₁ is selected from

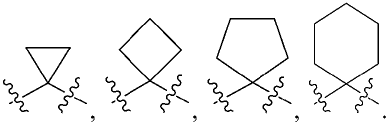

3. The compound according to claim 1 or 2, wherein, R₂ is selected from C₁~C₆ alkyl or deuterated C₁~C₆ alkyl;
preferably, R₂ is selected from methyl, ethyl or deuterated methyl.

4. The compound according to any one of claims 1~3, wherein, R₃ is selected from hydrogen or C₁~C₆ alkyl;
m, n are each independently selected from 0, 1 or 2;
preferably, 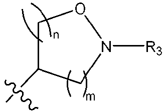 is selected from 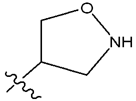 or

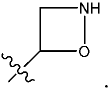

5. The compound according to any one of claims 1~4, wherein, X is O, Y is absent, or Y is O, X is absent.

6. The compound according to any one of claims 1~5, wherein, the compound of formula (I) is the compound represented by formula (IIa) or formula (IIb): 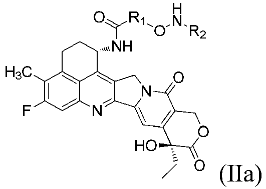
in formula (IIa), R₁, R₂ are each as defined in the compound of formula (I);
preferably, R₁ is selected from -CH₂-, -CD₂-, -(CH₂)₂-, -CH(CH₃)- or 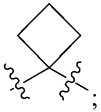
preferably, R₂ is selected from methyl, ethyl or deuterated methyl; 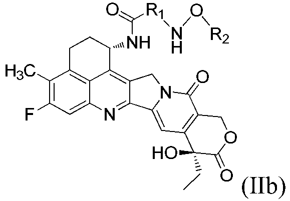
in formula (IIb), R₁, R₂ are each as defined in the compound of formula (I), preferably, R₁ is selected from -CH₂-, -CD₂-, -(CH₂)₂-, -CH(CH₃)- or 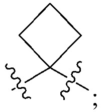
preferably, R₂ is selected from methyl, ethyl or deuterated methyl.

7. The compound, or pharmaceutically acceptable salt, stereoisomer, or prodrug thereof according to any one of claims 1~6, the compound of formula (I) is the compound represented by formulas (D1)~(D5):

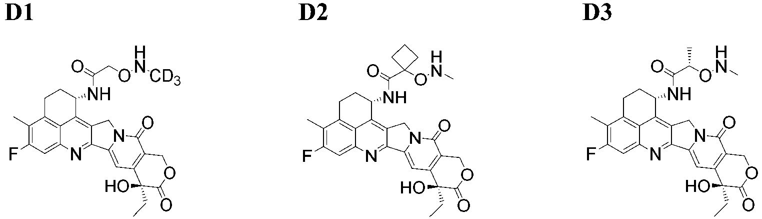

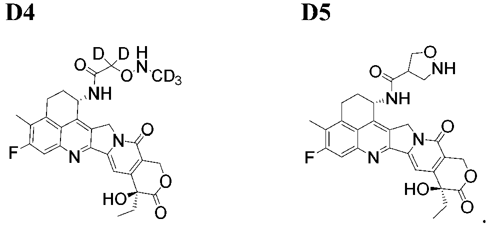

8. An antibody-drug conjugate, comprising a small molecule drug, a linker, and an antibody, wherein, the small molecule drug comprises the compound, or pharmaceutically acceptable salt, stereoisomer, or prodrug thereof according to any one of claims 1~7.

9. A pharmaceutical composition, comprising the compound, or pharmaceutically acceptable salt, stereoisomer, or prodrug thereof according to any one of claims 1~7, and a pharmaceutically acceptable excipient, or the antibody-drug conjugate according to claim 8.

10. Use of the compound, or pharmaceutically acceptable salt, stereoisomer, or prodrug thereof according to any one of claims 1~7, the antibody-drug conjugate according to claim 8, or the pharmaceutical composition according to claim 9 in the manufacture of a medicament for treating a cancer;
preferably, the cancer comprises any one or more of esophageal adenocarcinoma, gastric cancer, esophageal cancer, lung cancer, lung squamous cell carcinoma, breast cancer, breast adenocarcinoma and bladder cancer.

11. A method for treating a cancer, comprising the step of administering to a patient in need thereof a therapeutically effective amount of the compound, or pharmaceutically acceptable salt, stereoisomer, or prodrug thereof according to any one of claims 1~7, the antibody-drug conjugate according to claim 8, or the pharmaceutical composition according to claim 9;
preferably, the cancer comprises any one or more of esophageal adenocarcinoma, gastric cancer, esophageal cancer, lung cancer, lung squamous cell carcinoma, breast cancer, breast adenocarcinoma and bladder cancer.
